# EUROPEAN PATENT APPLICATION

(11) **EP 4 260 790 A1**
(43) Date of publication of application: **18.10.2023**
(21) Application number: 20965052.2
(22) Date of filing: 09.12.2020
(51) Int. Cl.: A61B 1/00

(54) **CONNECTION MECHANISM AND LOCK MECHANISM USED IN OPTICAL TOMOGRAPHY APPARATUS**

(71) Applicant: Genesis Medtech Japan Co., Ltd., Musashino-shi, Tokyo 180-0022 (JP)
(72) Inventor: SUZUKI, Yuji, Nagoya-shi, Aichi 457-0078 (JP); ITO, Takeshi, Nagoya-shi, Aichi 457-0078 (JP); TAIRA, Kenji, Musashino-shi, Tokyo 180-0022 (JP)
(74) Representative: MFG Patentanwälte Meyer-Wildhagen Meggle-Freund Gerhard PartG mbB
(86) International application number: PCT/JP2020/045793
(87) International publication number: WO 2022/123669

(57) **Abstract**

A connection mechanism (242) of the present invention is configured such that, in a state where an inclined end surface (536) of a male connection portion (500) is brought into contact with a protruding portion (618) of a female connection portion (600), by moving the male connection portion (500) toward the female connection portion (600), the male connection portion (500) rotates and the protruding portion (618) is inserted into a guide portion (534) and a connector (532) is connected to an adapter (610). The lock mechanism (243) is configured such that the protrusion (704) of the male lock portion (700) comes in contact with a wall (858) of a circumferential recess (842) disposed in a proximal cylindrical member (806) of the female lock portion (800), and in this state, the proximal cylindrical member (806) is further moved toward a proximal fixing member (804) against an urging force of a spring (854), and then the male lock portion (700) is rotated to move to a space (856) of the circumferential recess (842).

## Description

### Technical Field

The present invention relates to a connection mechanism and a lock mechanism used in an optical tomographic imaging apparatus.

### Background Art

A tomography system captures tomographic images of various biological tubular elements including biological tissue, such as digestive tract, pancreaticobiliary duct, fallopian tube, urethra, trachea, blood vessels, and lymphatic vessels. The tomography system generally includes a tomography system main body having an optical probe inserted into the biological tubular elements and a drive portion that rotates the optical probe to obtain tomographic images of entire circumferences of the biological tubular elements.

The optical probe is a replacement component attached to the main body of the tomography system every time of tomography. This optical probe needs to be removed from the main body of the tomography system and discarded after the tomography is performed. For this reason, for example, Patent Literature 1 and Patent Literature 2 propose a connection mechanism that enables replacement of the optical probe.

### Citation list

### Patent Literature

Patent Literature 1: JP 5139298 B2
Patent Literature 2: JP 5150725 B2

The connection mechanism is disposed in each of the optical probe and the drive portion. Each of the connection mechanisms of the optical probe and the drive portion has a connector through which an optical fiber passes and is configured to be separable. By fitting the connectors to each other, the optical fibers are coupled, and the optical probe and the drive portion are connected to each other.

On the other hand, if the connectors cannot be appropriately fitted into each other, the optical probe and the drive portion cannot be appropriately connected to each other. Therefore, it is preferable that the connection mechanisms of the optical probe and the drive portion are configured so that the connectors are guided to appropriate positions. In addition, it is preferable that the connection between the optical probe and the drive portion is locked to prevent the connection between the optical probe and the drive portion from being unintentionally released due to the rotation of the drive portion.

### Summary of Invention

### Technical Problem

Therefore, the present invention provides a connection mechanism and a lock mechanism that reliably connect the optical probe and the drive portion.

### Solution to Problem

In order to achieve this object, a connection mechanism according to an embodiment of the present invention is a connection mechanism that connects an optical probe used in an optical tomographic imaging apparatus and a drive portion that rotates the optical probe,
(a) the connection mechanism comprising:
   a female connection portion having an approximately cylindrical shape disposed on a distal end side of the drive portion; and
   a male connection portion having an approximately cylindrical shape disposed on a proximal end side of the optical probe,
(b) wherein the female connection portion includes
   a proximal rotation cylindrical portion having an approximately cylindrical shape disposed at a distal end of the female connection portion coaxially with a central axis of the female connection portion,
   an adapter fixed inside the female connection portion, and
   a protruding portion disposed on an inner circumferential surface of the female connection portion,
(c) wherein the male connection portion includes
   a distal rotation cylindrical portion having an approximately cylindrical shape disposed at a proximal end of the male connection portion coaxially with a central axis of the male connection portion,
   a connector fixed inside the distal rotation cylindrical portion correspondingly to the adapter,
   a guide portion disposed on an outer circumferential surface of the distal rotation cylindrical portion and extending from an end portion of a proximal end of the distal rotation cylindrical portion toward a distal end along the central axis of the male connection portion, and
   an inclined end surface that is symmetrical with respect to a plane including the guide portion and the central axis and extends toward a proximal end along the central axis of the male connection portion at a proximal end of the distal rotation cylindrical portion,
   the distal rotation cylindrical portion being configured to rotate about the central axis of the male connection portion, and
(d) wherein the male connection portion is rotated by moving the male connection portion toward the female connection portion in a state where the distal rotation cylindrical portion of the male connection portion is inserted into the proximal rotation cylindrical portion of the female connection portion and the inclined end surface of the male connection portion is brought into contact with the protruding portion of the female connection portion, the protruding portion is inserted into the guide portion, and the connector is connected to the adapter.

A lock mechanism according to another embodiment of the present invention is a lock mechanism that holds a connection between an optical probe used in an optical tomographic imaging apparatus and a drive portion that rotates the optical probe,
(a) the lock mechanism comprising:
   a female lock portion having an approximately cylindrical shape disposed on a distal end side of the drive portion; and
   a male lock portion having an approximately cylindrical shape disposed on a proximal end side of the optical probe, the male lock portion being configured to be inserted into the female lock portion,
(b) wherein the male lock portion includes a first protrusion disposed on an outer circumferential surface of the male lock portion,
(c) wherein the female lock portion includes
   a distal cylindrical member having a substantially cylindrical shape fixed to the drive portion coaxially with a central axis of the female lock portion at a distal end of the female lock portion,
   a proximal fixing member having an approximately cylindrical shape fixed to the drive portion to face the distal cylindrical member at a proximal end of the female lock portion,
   a proximal cylindrical member having an approximately cylindrical shape disposed between the distal cylindrical member and the proximal fixing member, the proximal cylindrical member being movable along the central axis of the female lock portion, and
   a spring that urges the proximal cylindrical member toward a distal end,

   wherein the distal cylindrical member includes
      a protrusion disposed on an inner circumferential surface of the distal cylindrical member and extending toward a proximal end of the female lock portion along the central axis of the female lock portion, and
      a first longitudinal groove disposed on one end in a circumferential direction of the protrusion, the first protrusion of the male lock portion passing through the first longitudinal groove,
   wherein the proximal cylindrical member includes a circumferential recess having a shape corresponding to the protrusion on an inner circumferential surface of the proximal cylindrical member and having a circumferential length longer than the protrusion,
   the circumferential recess being configured to accommodate the protrusion in a state where the proximal cylindrical member being urged by the spring and form a space on a side opposing the first longitudinal groove across the protrusion, and
(d) wherein the first protrusion of the male lock portion is configured to, when the male lock portion is inserted into the distal cylindrical member of the female lock portion, pass through the first longitudinal groove of the distal cylindrical member, come in contact with a wall of the circumferential recess disposed in the proximal cylindrical member of the female lock portion, further, in this state, move the proximal cylindrical member toward the proximal fixing member against an urging force of the spring, and then rotate the male lock portion to move to the space of the circumferential recess.

### Advantageous Effects of Invention

An embodiment of the present invention provides a connection mechanism in which a connector disposed in a male connection portion of an optical probe and an adapter disposed in a female connection portion of a drive portion are reliably guided to appropriate positions, and thus the optical probe and the drive portion are reliably connected. Further, another embodiment of the present invention provides a lock mechanism in which a male lock portion of an optical probe and a female lock portion of a drive portion locks a connection between a male connection portion of the optical probe and a female connection portion of the drive portion to prevent a connection between the optical probe and the drive portion from being unintentionally released.

### Brief Description of Drawings

FIG. 1 illustrates a schematic configuration of an optical tomographic imaging device according to an embodiment of the present invention.
FIG. 2 is a schematic view illustrating a male connection portion and a male lock portion.
FIG. 3 is a cross-sectional view of the male connection portion and the male lock portion illustrated in FIG. 2.
FIG. 4 is a schematic view illustrating a connection between the male connection portion and a female connection portion.
FIG. 5 is a schematic view illustrating the connection between the male connection portion and the female connection portion.
FIG. 6 is a schematic view illustrating the connection between the male connection portion and the female connection portion.
FIG. 7 is a schematic view illustrating a female lock portion.
FIG. 8 is a schematic view illustrating fitting between the male lock portion and the female lock portion.
FIG. 9 is a schematic view illustrating the fitting between the male lock portion and the female lock portion.
FIG. 10 is a schematic view illustrating the fitting between the male lock portion and the female lock portion.
FIG. 11 is a schematic view illustrating the fitting between the male lock portion and the female lock portion. Description of Embodiments

Hereinafter, an embodiment of a connection mechanism and a lock mechanism used in an optical tomographic imaging device according to the present invention will be described with reference to the accompanying drawings.

### [Optical Tomographic Imaging Device]

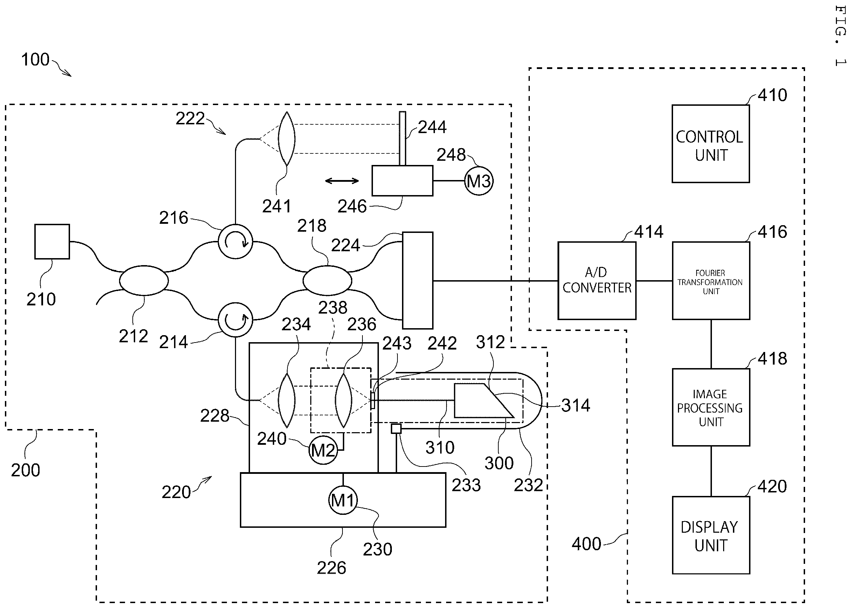

FIG. 1 illustrates an outline of a wavelength sweep type optical tomographic imaging device (Swept Source Optical Coherence Tomography (SS-OCT)) 100 according to the embodiment.

The SS-OCT 100 includes an optical unit 200 and a signal processing unit 400.

### [Optical Unit]

The optical unit 200 includes a plurality of optical elements. The plurality of optical elements includes a wavelength swept light source 210, a first optical coupler 212, a first optical circulator 214, a second optical circulator 216, a second optical coupler 218, a biological tubular element imaging unit 220, an optical distance adjustment unit 222, and a detection unit 224. In these optical elements, associated elements are optically coupled to each other by optical transfer elements, such as optical fibers, as described below.

### [Wavelength Swept Light Source]

The wavelength swept light source 210 outputs light necessary for imaging a cross section of a biological tubular element. The wavelength swept light source 210 can periodically change a wavelength of output light, and is configured to sweep a wavelength from 1260 nm to 1360 nm at a frequency of 100 kHz, for example.

### [First Optical Coupler]

The first optical coupler (separator) 212 is a portion optically coupled by partially heating to fuse two optical fibers arranged in parallel. One of the two optical fibers optically couples the first optical coupler 212 and the first optical circulator 214, and the other optical fiber optically couples the wavelength swept light source 210 and the second optical circulator 216 with the first optical coupler 212 being interposed therebetween. Therefore, the light output from the wavelength swept light source 210 is split into two light beams by the first optical coupler 212, one of the split light beams is sent to the first optical circulator 214, and the other split light beam is sent to the second optical circulator 216.

### [First Optical Circulator]

The first optical circulator 214 is a 3-port optical circulator, in which a first port is connected to the first optical coupler 212, a second port is connected to the second optical coupler 218, and a third port is connected to the biological tubular element imaging unit 220. The first optical circulator 214 is configured to send light sent from the wavelength swept light source 210 to the first optical circulator 214 through the first optical coupler 212 to the biological tubular element imaging unit 220, and send light returned from the biological tubular element imaging unit 220 to the second optical coupler 218.

### [Second Optical Circulator]

The second optical circulator 216 is a 3-port optical circulator, in which a first port is connected to the first optical coupler 212, a second port is connected to the second optical coupler 218, and a third port is connected to the optical distance adjustment unit 222. The second optical circulator 216 is configured to send light sent from the wavelength swept light source 210 to the second optical circulator 216 through the first optical coupler 212 to the optical distance adjustment unit 222, and send light returned from the optical distance adjustment unit 222 to the second optical coupler 218.

### [Second Optical Coupler]

The second optical coupler 218 (interference unit) is a portion optically coupled by heating to fuse some midpoint of an optical fiber having one end (proximal end) connected to the first optical circulator 214 and some midpoint of an optical fiber having one end (proximal end) connected to the second optical circulator 216. The second optical coupler 218 obtains interference light by superimposing light (reflected light) sent from the biological tubular element imaging unit 220 to the second optical coupler 218 through the first optical circulator 214 on light (reference light) sent from the optical distance adjustment unit 222 to the second optical coupler 218 through the second optical circulator 216.

### [Biological Tubular Element Imaging Unit]

The biological tubular element imaging unit 220 includes a base 226 and a linear motion portion 228 that linearly moves in a predetermined direction (left-right direction in FIG. 1) with respect to the base 226. The linear motion portion 228 is coupled to a linear motion motor 230 disposed on the base 226, and is configured to move forward or backward in a predetermined direction based on driving of the linear motion motor 230. A proximal end of a hollow cylindrical flexible tube (hereinafter, referred to as a "sheath") 232 made of a translucent resin is detachably fixed to the base 226 by a holder 233. As illustrated in the drawing, the sheath 232 is open at the proximal end and closed at the distal end.

The linear motion portion 228 is provided with a proximal collimation lens 234 and a distal collimation lens 236. The proximal collimation lens 234 and the distal collimation lens 236 are aligned with each other on one optical axis with constant spacing, and light passing through the proximal collimation lens 234 passes on the optical axis of the distal collimation lens 236.

The proximal collimation lens 234 is fixed to the linear motion portion 228 and is optically coupled to the first optical circulator 214 via the optical fiber.

The distal collimation lens 236 is supported to a rotation portion (drive portion) 238 disposed in the linear motion portion 228. The rotation portion 238 is supported to the linear motion portion 228 to be rotatable about the optical axes of the proximal collimation lens 234 and the distal collimation lens 236 with the distance between the proximal collimation lens 234 and the distal collimation lens 236 being kept constant.

The rotation portion 238 is drivingly coupled to a rotary motor 240 fixed to the linear motion portion 228 with a rotation transmission mechanism (not illustrated) including, for example, a gear and a toothed belt being interposed therebetween.

An optical probe 300 is detachably coupled to the rotation portion 238. The optical probe 300 includes an optical fiber 310 and an optical component 312. The optical component 312 is coupled to the distal end of the optical fiber 310. A proximal end of the optical fiber 310 is detachably coupled to the rotation portion 238 with a connection mechanism 242 and a lock mechanism 243, described below, being interposed therebetween. The optical fiber 310 rotates together with the rotation of the rotation portion 238, and light collected by the distal collimation lens 236 enters a core of the optical fiber 310 through the proximal end.

The light that has entered the optical fiber 310 through the proximal end enters the optical component 312 via the distal end of the optical fiber 310. The optical component 312 has an inclined surface 314 at the distal end. The light that has entered the optical component 312 is reflected by the inclined surface 314, then travels in a direction orthogonal to the optical fiber 310, and is emitted in a radial direction from a side surface of the optical component 312 at the distal end. Conversely, the light that has entered the optical component 312 from the side surface of the optical component 312 at the distal end enters the optical fiber 310 via the inclined surface 314.

The sheath 232 and the optical probe 300 described above are provided in a state where the optical probe 300 is inserted into the sheath 232. In use, the optical fiber 310 of the optical probe 300 is coupled to the rotation portion 238 with the connection mechanism 242 and the lock mechanism 243 being interposed therebetween, and a proximal end of the sheath 232 is fixed to the base 226 by the holder 233.

Therefore, the linear motion portion 228 and the optical probe 300 move forward and backward with respect to the base 226 based on the driving of the linear motion motor 230. On the other hand, the sheath 232 is held to the base 226. Therefore, the optical probe 300 moves forward and backward in the sheath 232 based on the driving of the linear motion motor 230. Further, the rotation portion 238 rotates about the optical axis based on the driving of the rotary motor 240. At this time, since the optical probe 300 is coupled to the rotation portion 238 with the connection mechanism 242 and the lock mechanism 243 being interposed therebetween, the optical probe rotates together with the rotation portion 238. As a result, the light sent from the first optical circulator 214 to the biological tubular element imaging unit 220 enters the optical fiber 310 via the proximal collimation lens 234 and the distal collimation lens 236. Thereafter, the light is emitted from the optical fiber 310 in a radial direction via the optical component 312, and the emitted light is scanned at a constant speed in a circumferential direction around the optical axis.

The sizes of the sheath 232 and the optical probe 300 accommodated in the sheath 232 are appropriately determined in accordance with the size of the biological tubular element to be imaged. In order to stably rotate the optical probe 300 in the sheath 232, an inner diameter of the sheath 232 and an outer diameter of the optical probe 300 accommodated therein are determined to be larger than the maximum outer diameter of the optical component 312 by, for example, about 50 um. For example, in a case where the maximum outer diameter of the optical component 312 is 200 um to 500 µm, the inner diameter of the sheath 232 is about 250 um to 550 µm.

### [Optical Distance Adjustment Unit]

The optical distance adjustment unit 222 includes a collimation lens 241 and a reference mirror 244. The collimation lens 241 is immovably fixed. The reference mirror 244 has a reflection surface (mirror surface) on the optical axis of the collimation lens 241. The reflection surface is perpendicular to the optical axis. The reference mirror 244 is supported to the linear motion portion 246. The linear motion portion 246 is movable along the optical axis of the collimation lens 241. The linear motion portion 246 is also coupled to a linear motion motor 248, and is configured to advance and retract toward the collimation lens 241 based on the driving of the linear motion motor 248, thereby adjusting an optical distance of the reference light (optical distance).

### [Detection Unit]

The detection unit 224 is an optical component that receives light transmitted from the second optical coupler 218 and photoelectrically converts the light. In particular, the detection unit is a dual balanced detector having two light input units. The dual balanced detector includes two photodiodes that detect light (interference light) transmitted from the second optical coupler 218, and these two photodiodes are connected to distal ends of two optical fibers constituting the second optical coupler 218. The detection unit 224 is also configured to convert light input from the two optical fibers into electric signals, and then cancel direct current (DC) components included in the electric signals to extract only electric signals based on the interference light.

### [Signal Processing Unit]

The signal processing unit 400 includes a controller 410, an analog-digital (A/D) conversion unit 414, a Fourier transformation unit 416, an image processing unit 418, and an image display unit (monitor) 420. On the drawing, the A/D conversion unit 414, the Fourier transformation unit 416, and the image processing unit 418 are illustrated as functional blocks, and do not need to have a physical configuration. Thus, they may be some parts of a program installed in a storage unit of the controller 410 described later.

### [Controller]

Although not illustrated, the controller 410 includes a control unit, an arithmetic unit, and a storage unit. The storage unit can temporarily store a program necessary for executing processing described later and various data (for example, image data) to be generated in the course of the processing described later. The arithmetic unit executes an arithmetic operation to be executed in the course of processing described later, in accordance with a program stored in the storage unit.

Although not illustrated, the controller 410 is communicably connected to various devices (the wavelength swept light source 210, the rotary motor 240, the linear motion motor 230, and the linear motion motor 248 of the reference mirror 244) included in the optical unit 200, and is configured to individually drive and control these devices in accordance with the program in the storage unit.

Although not illustrated, the controller 410 is connected to the input unit, and is configured to execute processing described below, based on a signal input from the input unit. The input unit may be any of a keyboard, a pointing device, a touch screen, a mouse, a joystick, a trackball, a scanner, an optical character reader (OCR), an optical mark reader (OMR), an audio input device, a tablet, and the like.

### [A/D Conversion Unit]

The A/D conversion unit 414 converts an analog signal (an electric signal based on interference light) output from the detection unit 224 into a digital signal.

### [Fourier Transformation Unit]

The Fourier transformation unit 416 performs Fourier transformation (for example, fast Fourier transformation or discrete Fourier transformation) on the digital signal output from the A/D conversion unit 414 to obtain the intensity (power spectrum) of the interference light with respect to a difference (optical distance difference) between an optical distance of the reflected light and an optical distance of the reference light.

### [Image Processing Unit]

The image processing unit 418 receives a power spectrum including spectral components of the biological tubular element and the sheath 232 from the Fourier transformation unit 416, and outputs image information for each rotation angle of the optical probe 300 in accordance with intensity distributions of the biological tubular element and the sheath 232 in the power spectrum.

### [Image Display Unit]

The image display unit 420 is a general monitor, and outputs an image output from the image processing unit 418.

### [Connection Mechanism]

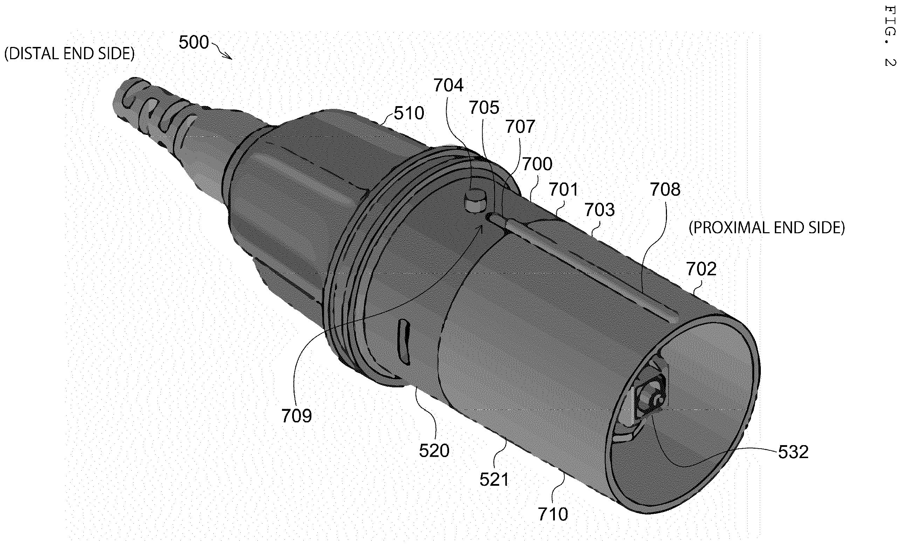

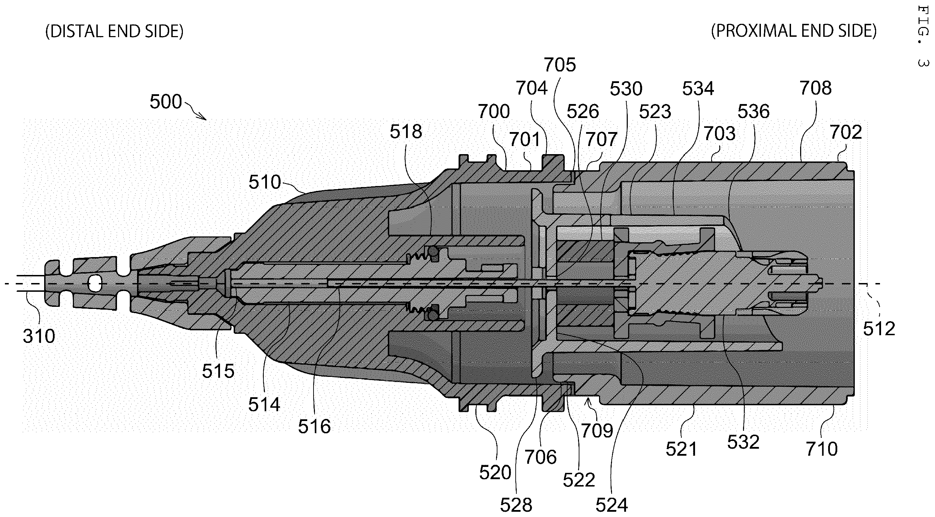

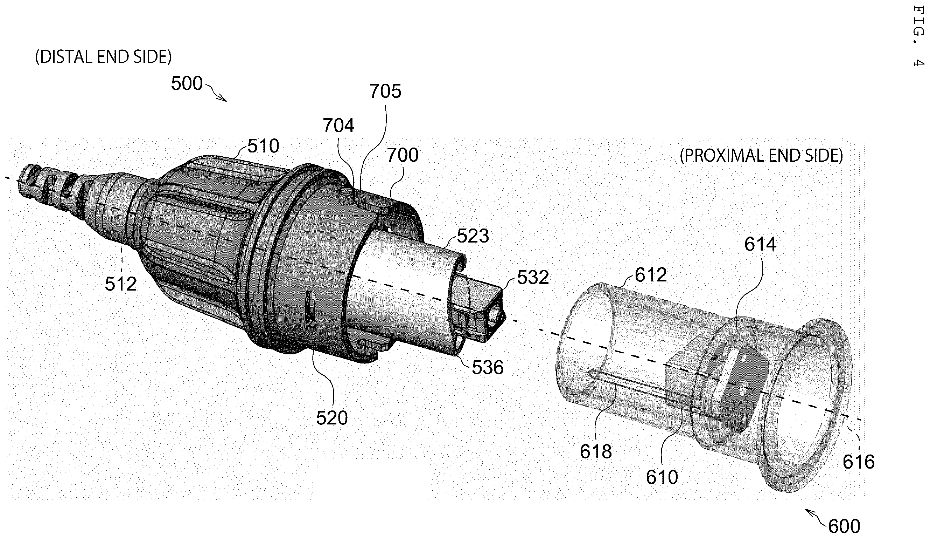

The connection mechanism 242 that connects the optical probe 300 and the rotation portion 238 includes a male connection portion 500 (see FIGS. 2 and 3) disposed in the optical probe 300 and a female connection portion 600 disposed in the rotation portion 238 (see FIG. 4).

### [Male Connection Portion on Optical Probe Side]

FIGS. 2 and 3 illustrate the male connection portion 500. The male connection portion 500 includes a main shaft portion 510. The main shaft portion 510 includes a hollow cylindrical fiber holding portion 514 extending along a central axis 512. The fiber holding portion 514 has a waveguide (fiber insertion hole) 515 including an elongated hole extending along the central axis 512. The proximal end of the optical fiber 310 is optically coupled to the distal end (the left end portion in FIG. 3) of the waveguide 515.

An optical fiber 516 is accommodated in the waveguide 515 to be rotatable about the axis 512 and movable along the axis 512. The inner diameter of the waveguide 515 is designed to be approximately equal to the outer diameter of the optical fiber 516. Therefore, the light emitted from the optical fiber 516 to the waveguide 515 enters the optical fiber 310 via the waveguide 515, and the light emitted from the optical fiber 310 to the waveguide 515 enters the optical fiber 516. As described above, the waveguide 515 has a function of optically coupling the optical fiber 310 at the distal end and the optical fiber 516 at the proximal end.

The main shaft portion 510 includes two cylindrical portions protruding toward the proximal end (right side in FIGS. 2 and 3), that is, an inner cylindrical portion 518 and an outer cylindrical portion (distal cylindrical cover) 520. The inner cylindrical portion 518 is disposed concentrically with the central axis 512 so as to surround the proximal end of the fiber holding portion 514. The outer cylindrical portion 520 is disposed concentrically with the central axis 512 and the inner cylindrical portion 518 so as to surround the inner cylindrical portion 518. The proximal end of the outer cylindrical portion 520 extends toward the proximal end side with respect to the proximal end of the inner cylindrical portion 518, and a proximal cylindrical cover 521 is concentrically connected to the extended portion. A small-diameter cylindrical portion 522 is formed on the distal end side of the proximal cylindrical cover 521. The small-diameter cylindrical portion 522 is inserted into the proximal end of the outer cylindrical portion 520 to couple the distal and proximal cylindrical covers 520 and 521 to each other.

The male connection portion 500 on the optical probe 300 side includes, at the proximal end, a distal rotation cylindrical portion 523 having an approximately cylindrical shape that extends along the central axis 512 and is disposed coaxially with the central axis 512 so as to be rotatable about the axis 512. The proximal end of the distal rotation cylindrical portion 523 is open. An annular inner wall 524 extending radially inward and an annular outer wall (flange) 528 extending radially outward are integrally formed on the distal end side of the distal rotation cylindrical portion 523. A through hole 526 whose center is the axis 512 is formed at the center of the annular inner wall 524. The through hole 526 has a size that allows the optical fiber 516 to pass therethrough. The outer diameter of the annular outer wall 528 is smaller than the inner diameter of the outer cylindrical portion 520 and larger than the inner diameter of the small-diameter cylindrical portion 522. Therefore, the distal rotation cylindrical portion 523 can move between a retracted position where the distal end surface of the annular inner wall 524 comes in contact with the proximal end of the inner cylindrical portion 518 and an advanced position where the proximal end surface of the annular outer wall 528 comes in contact with the small-diameter cylindrical portion 522.

Inside the distal rotation cylindrical portion 523, an elastic member 530 having a hollow cylindrical shape fixed to the annular inner wall 524 and a fiber connector 532 fixed to the proximal end of the elastic member 530 are disposed concentrically with the central axis 512.

The elastic member 530 is preferably made of natural rubber or synthetic rubber such as nitrile rubber.

The fiber connector 532 is, for example, a subscriber (SC) connector used for connection of an optical fiber, and is optically connected with the proximal end of the optical fiber 516. The proximal end of the fiber connector 532 has a shape and a size such that it can be connected to an adapter (drive portion-side adapter) 610 of the female connection portion 600 described later.

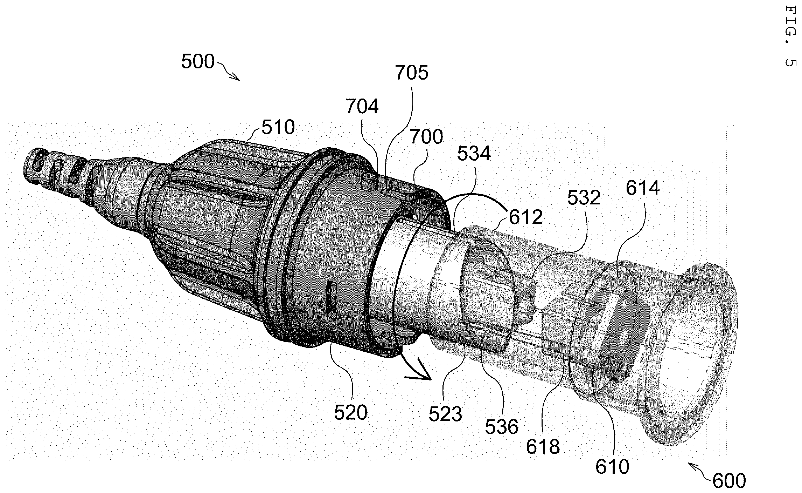

As illustrated in FIGS. 3 and 5, the distal rotation cylindrical portion 523 includes a guide portion 534 having a predetermined length extending in parallel with the axis 512 from the proximal end toward the distal end of the distal rotation cylindrical portion 523. In the embodiment, the guide portion 534 is a slot penetrating the inner circumferential surface and the outer circumferential surface of the distal rotation cylindrical portion 523, but may be a bottomed groove formed along the inner circumferential surface of the distal rotation cylindrical portion 523.

An inclined end surface 536 along a surface obliquely intersecting with the axis 512 is formed at the proximal end of the distal rotation cylindrical portion 523. A portion (deepest portion) located on the most distal end side of the inclined end surface 536 is aligned with the guide portion 534, and the inclined end surface 536 has a symmetrical shape with respect to a plane including the guide portion 534 and the axis 512.

### [Female Connection Portion on Rotation Portion Side]

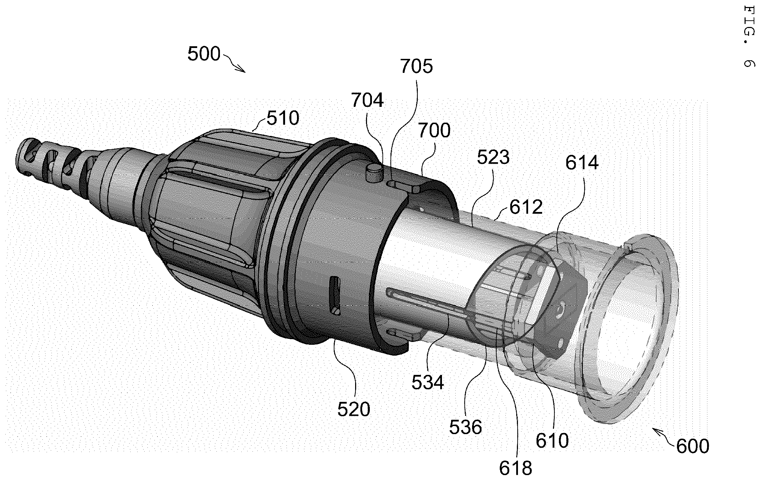

FIGS. 4 to 6 illustrate the female connection portion 600. The female connection portion 600 includes a proximal rotation cylindrical portion 612. The proximal rotation cylindrical portion 612 constituting a part of the connection mechanism 242 is disposed coaxially with the optical axis of the distal collimation lens 236 disposed in the rotation portion 238, and is coupled to the rotation portion 238 so as to rotate together with the rotation portion 238. The proximal rotation cylindrical portion 612 is also opened at the proximal end and the distal end. The adapter 610 having a shape and a size connectable to the connector 532 is fixed to the distal end of an annular wall 614 disposed between the proximal end and the distal end. The central axis of the adapter 610 is aligned with a central axis 616 of the proximal rotation cylindrical portion 612. A light guide path (not illustrated) formed inside the adapter 610 is optically coupled to the distal collimation lens 236 disposed in the rotation portion 238.

The proximal rotation cylindrical portion 612 has an inner diameter approximately equal to the outer diameter of the distal rotation cylindrical portion 523 of the male connection portion 500 on the optical probe 300 side, and is configured to be exteriorly mounted to the distal rotation cylindrical portion 523 with the male connection portion 500 and the female connection portion 600 being coupled to each other. The inner circumferential surface of the proximal rotation cylindrical portion 612 is provided with an elongated ridge 618 or a protruding portion including a columnar protrusion extending straight along the central axis 616 correspondingly to the guide portion 534 of the distal rotation cylindrical portion 523. By inserting the ridge 618 of the proximal rotation cylindrical portion 612 into the guide portion 534 of the distal rotation cylindrical portion 523, the proximal rotation cylindrical portion 612 is positioned in the circumferential direction with respect to the distal rotation cylindrical portion 523, and the connector 532 and the adapter 610 can be connected.

### [Lock Mechanism]

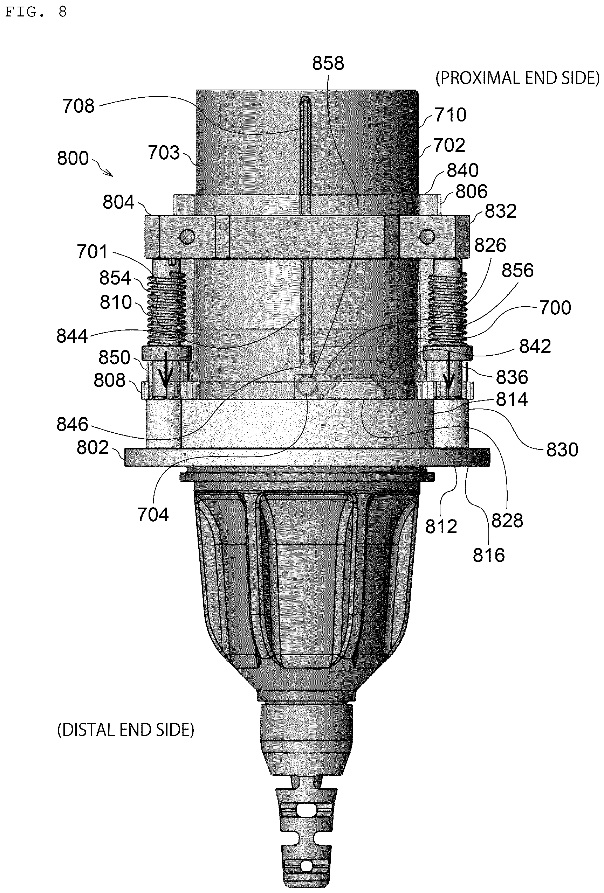

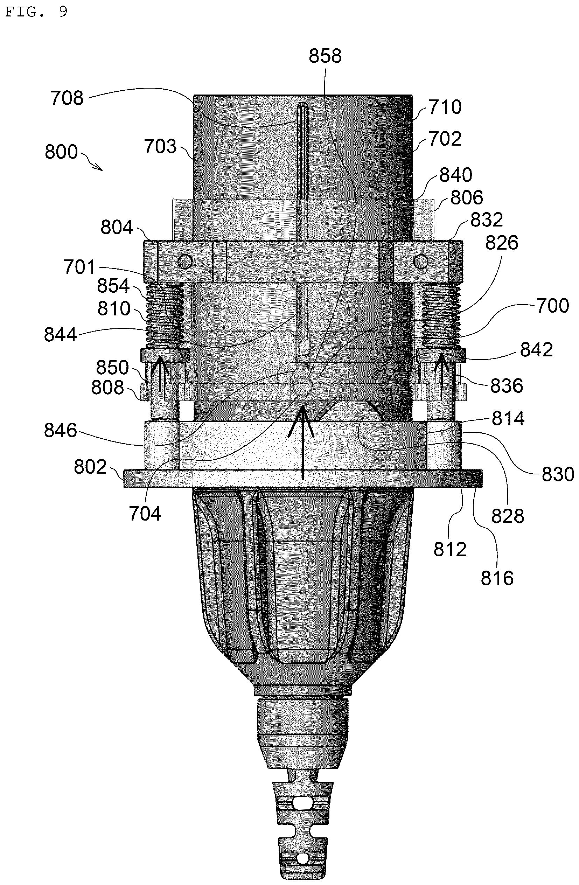

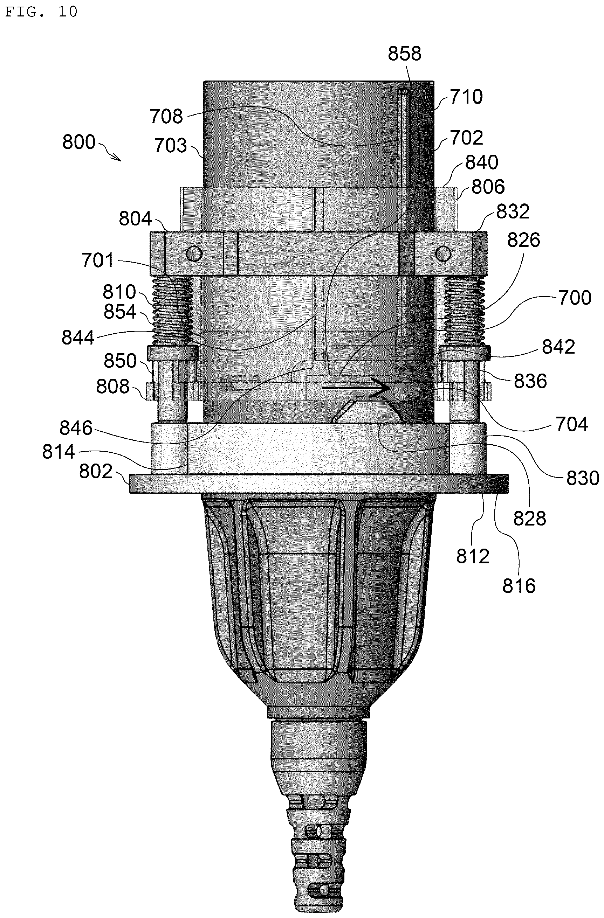

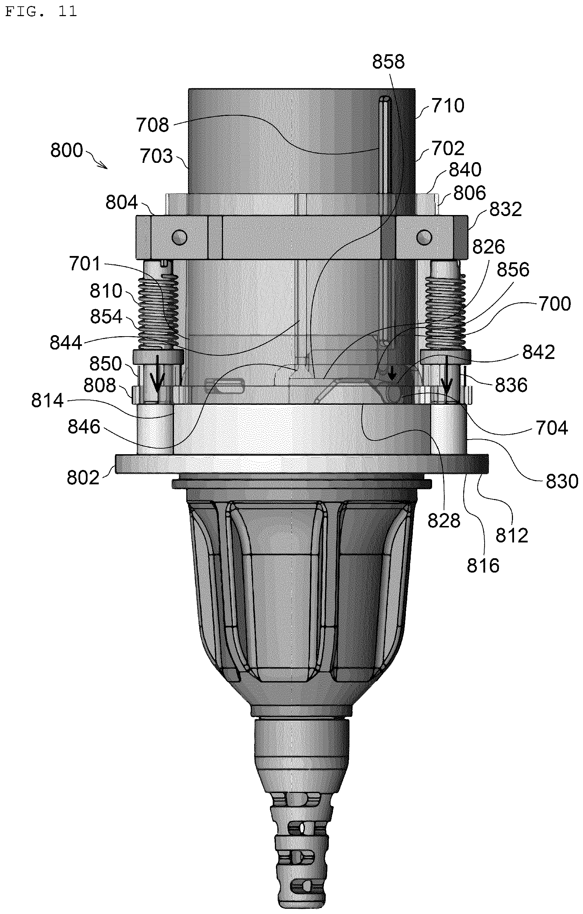

In the embodiment, the lock mechanism 243 that holds the connection between the male connection portion 500 of the optical probe 300 and the female connection portion 600 of the rotation portion 238 includes a male lock portion 700 on the optical probe 300 side and a female lock portion 800 on the rotation portion 238 side (see FIGS. 2, 3, and 7 to 11).

### [Male Lock Portion]

As illustrated in FIGS. 2 and 3, the male lock portion 700 has a cylindrical outer circumferential surface 701 and a cylindrical outer circumferential surface 702 of the proximal cylindrical cover 521, provided on the proximal end of the outer cylindrical portion (distal cylindrical cover) 520 constituting a part of the male connection portion 500. The outer circumferential surfaces 701 and 702 have equal outer diameters and are disposed coaxially with the axis 512 to form a cylindrical outer circumferential surface 703 continuous in the axial direction.

On the outer circumferential surface 701, a pair of columnar protrusions 704 (only one protrusion is illustrated) protruding outward in the radial direction is formed in symmetrical positions with respect to the central axis 512 (spaced at 180 degrees in the circumferential direction) so as to be placed some distance away from the proximal end of the outer circumferential surface 701. At the proximal end of the outer circumferential surface 701, slots 705 penetrating the outer cylindrical portion 520 are formed at symmetrical positions with respect to the central axis 512 (spaced at 180 degrees in the circumferential direction) respectively along surfaces including the central axis 512 and the centers of the protrusions 704. On the other hand, a short ridge 707 extending along the central axis 512 is formed on the outer circumferential surface 706 of the small-diameter cylindrical portion 522 (see FIG. 3), and another long ridge 708 extending along the central axis 512 is formed on the outer circumferential surface 702. The ridges 707 and 708 are disposed in line with each other. A height of the ridge 707 is determined so that its distal end (radially outer end) is aligned with the outer circumferential surface 702 of a cover 710.

With such a configuration, the outer cylindrical portion (distal cylindrical cover) 520 and the proximal cylindrical cover 521 are coupled in a state that they are aligned with each other in the circumferential direction by fitting the ridges 707 of the proximal cylindrical cover 521 respectively into the slots 705. As described above, since the height of each ridge 707 is determined such that the distal end thereof is aligned with the outer circumferential surface 702, each ridge 707 does not protrude from the outer circumferential surface 701 in a state where the covers 520 and 521 are coupled. Further, a predetermined gap 709 (see FIG. 2) is provided between each protrusion 704 and each ridge 708 in the direction of the axis 512.

### [Female Lock Portion]

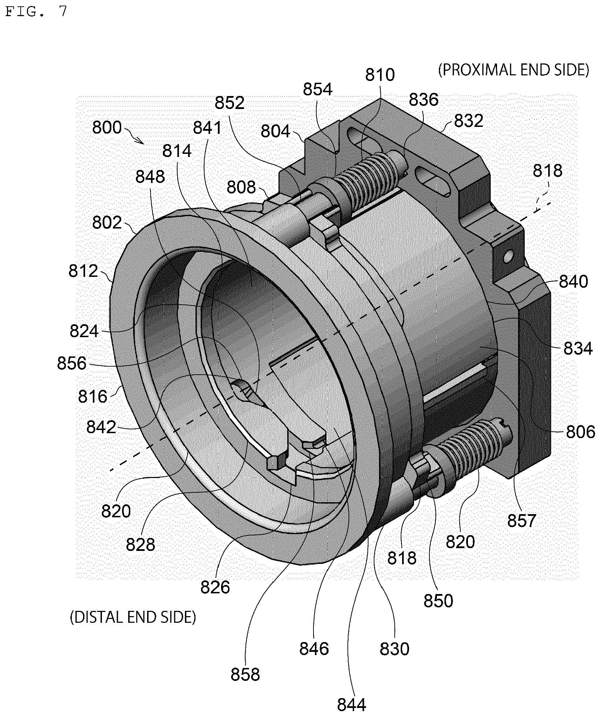

FIG. 7 illustrates an appearance of the female lock portion 800. The female lock portion 800 roughly includes a distal cylindrical member 802, a proximal fixing member 804, a proximal cylindrical member 806, a coupling mechanism 808, and an urging mechanism 810.

The distal cylindrical member 802 includes a distal cylindrical portion 812 and a proximal cylindrical portion 814 in an integral manner. The distal cylindrical portion 812 includes an annular flange 816 integrally protruding outward at the distal end of the distal cylindrical portion 812. The distal cylindrical portion 812 includes a large-diameter cylindrical inner surface 820 centered around a central axis 818. The proximal cylindrical portion 814 has a small-diameter cylindrical inner surface (not illustrated) centered around the central axis 818. An annular protruding portion 824 protruding inward is formed at a distal end of the small-diameter cylindrical inner surface. The inner diameter of the annular protruding portion 824 is approximately equal to the outer diameters of the outer circumferential surfaces 703 (see FIGS. 2 and 3) of the distal cylindrical cover 520 and the proximal cylindrical cover 521.

In the annular protruding portion 824, a pair of longitudinal grooves 826 extending along the central axis 818 is formed symmetrically with respect to the central axis 818 (only one groove is illustrated in the drawing). The depth (corresponding to the height of the annular protruding portion 824) and width of the longitudinal grooves 826 are approximately equal to the height and width of the protrusion 704 of the male lock portion 700. In the annular protruding portion 824, an approximately trapezoidal protrusion 828 is formed by protruding a portion adjacent to one side of each longitudinal groove 826 (a lower-stream side in a clockwise direction of the longitudinal groove 826 when viewed from the distal end toward the proximal end) from the proximal end. As illustrated in the drawing, corners on both sides in the circumferential direction of the trapezoidal protrusion 828 are inclined along an oblique straight line or a curve.

In the distal cylindrical member 802, three protruding portions 830 circumferentially disposed at regular gaps are formed on the proximal end of the flange 816. A coupling hole (not illustrated) extending along the axis 818 from the proximal end surface toward the distal end is formed in each protruding portion 830.

The proximal fixing member 804 includes an annular ring 832. The ring 832 has a through hole 834 having an inner diameter approximately equal to an outer diameter of the proximal cylindrical member 806 described later. Three coupling pins 836 circumferentially disposed at regular gaps are fixed to the distal end surface of the ring 832. The size and disposing of the coupling pins 836 correspond to the coupling holes (not illustrated) formed in the protruding portions 830 of the distal cylindrical member 802. Therefore, the distal cylindrical member 802 and the proximal fixing member 804 are coupled to each other with certain gaps being provided between the protruding portions 830 of the distal cylindrical member 802 by fitting the three coupling pins 836 into the corresponding protruding portions 830 of the distal cylindrical member 802.

The proximal cylindrical member 806 includes a hollow cylindrical body 840. The outer diameter of the hollow cylindrical body 840 is approximately equal to the inner diameter of the small-diameter cylindrical inner surface of the distal cylindrical member 802 and the inner diameter of the through hole 834 of the ring 832 in the proximal fixing member 804. Therefore, as illustrated in the drawing, the proximal cylindrical member 806 can be inserted into the small-diameter cylindrical inner surface of the distal cylindrical member 802 through the ring 832.

The inner circumferential surface 841 of the proximal cylindrical member 806 has an inner diameter approximately equal to the inner diameter of the annular protruding portion 824 formed on the proximal cylindrical portion 814 of the distal cylindrical member 802. Therefore, in a state where the distal end of the proximal cylindrical member 806 is inserted into the distal cylindrical member 802 from the proximal end (the state illustrated in FIG. 7), the inner circumferential surface 841 of the proximal cylindrical member 806 is located on the same cylindrical surface as the inner circumferential surface of the annular protruding portion 824.

By scraping a distal end portion of the inner circumferential surface 841 of the proximal cylindrical member 806 over a range of a predetermined circumferential length, a circumferential recess 842 is formed. The circumferential length of the circumferential recess 842 is larger than the circumferential length of the trapezoidal protrusion 828 of the distal cylindrical member 802. The depth (radial dimension) of the circumferential recess 842 is approximately equal to the depth (radial dimension) of the longitudinal groove 826, and is equal to or larger than the height (radial dimension) of the trapezoidal protrusion 828. The axial length of the circumferential recess 842 is approximately equal to the axial length of the trapezoidal protrusion 828.

A recess or a recessed surface 844 is formed in a region extending from a position away from the circumferential recess 842 toward the proximal end by a predetermined distance toward the proximal end on the inner circumferential surface 841 of the proximal cylindrical member 806. The recessed surface 844 has a circumferential width equal to the width of the circumferential recess 842, and an axial length approximately equal to the axial length of the longitudinal ridge 708 in the male lock portion 700. The depth (radial dimension) of the recessed surface 844 is smaller than the depth (radial dimension) of the longitudinal groove 826.

The circumferential recess 842 and the recessed surface 844 are coupled by a longitudinal groove 846 that couples first circumferential ends (clockwise upstream end portions of the circumferential recess 842 and the recessed surface 844 when viewed from the distal end side toward the proximal end) thereof. An island-shaped circumferential wall 848 is formed in a region surrounded on three sides by the circumferential recess 842, the recessed surface 844, and the longitudinal groove 846. The axial length or width of the circumferential wall 848 is equal to the axial length of the gap 709 between the protrusion and the ridge in the male lock portion 700. The depths of the recessed surface 844 and the longitudinal groove 846 are equal to each other, and are smaller than the height of the trapezoidal protrusion 828 and the depth of the longitudinal groove 826 (both are radial dimensions).

Three protruding portions 850 circumferentially disposed at constant intervals are fixed to the outer circumferential surface of the proximal cylindrical member 806. A through hole 852 is formed on each protruding portion 850 in the direction of the central axis 818. The coupling pins 836 of the proximal fixing member 804 are inserted respectively into the through holes 852 in a state where the distal cylindrical member 802, the proximal fixing member 804, and the proximal cylindrical member 806 are combined. The distal ends of the coupling pins 836 are fitted into the coupling holes (not illustrated) of the protruding portions 830, respectively. Further, a spring 854 is disposed on each coupling pin 836, and as illustrated in FIG. 7, the proximal cylindrical member 806 is urged to a position where it is moved to the most distal side with respect to the proximal fixing member 804.

The positions of the coupling holes of the protruding portions 830 and the through holes 852 of the protruding portions 850 are determined such that, in a state that the distal cylindrical member 802, the proximal fixing member 804, and the proximal cylindrical member 806 are combined, the distal surface of the proximal cylindrical member 806 comes in contact with the proximal surface of the annular protruding portion 824 by the urging forces of the springs 854, the longitudinal grooves 826 of the distal cylindrical member 802 and the longitudinal grooves 846 of the proximal cylindrical member 806 are respectively aligned with each other in the central axis direction, and the trapezoidal protrusion 828 is accommodated in the circumferential recesses 842. As described above, since the circumferential length of the circumferential recess 842 is longer than the circumferential length of the trapezoidal protrusion 828 of the distal cylindrical member 802, in the combined state, a space (protrusion accommodating chamber) 856 having a predetermined circumferential length is formed on a side opposing the longitudinal grooves 826 and 846 across the trapezoidal protrusion 828.

The outer circumferential surface of the proximal cylindrical member 806 is also provided with a rib 857 protruding radially outward on the proximal end side with respective to the protruding portions 850. The rib 857 extends along the axis 818, and is spaced apart from the proximal fixing member 804 by a certain distance such that the distance between the proximal end of the rib 857 and the proximal fixing member 804 is larger than the diameter of each protrusion 704.

In the female lock portion 800 configured as described above, the proximal fixing member 804 is fixed to the linear motion portion 228 using an appropriate coupling member such as a bolt in a state where the proximal rotation cylindrical portion 612 of the female connection portion 600 is accommodated inside the proximal cylindrical member 806. In this state, the proximal rotation cylindrical portion 612 of the female connection portion 600 is coupled to the rotation portion 238 of the linear motion portion 228 and is rotatable together with the rotation of the rotation portion 238.

In the above configuration, in a case where the optical probe 300 is connected to the biological tubular element imaging unit 220, the proximal end of the male connection portion 500 faces the female connection portion 600. In this state, the male lock portion 700 on the optical probe 300 side faces the female lock portion 800 of the linear motion portion 228, and the distal rotation cylindrical portion 523 of the male connection portion 500 on the optical probe 300 side faces the proximal rotation cylindrical portion 612 of the female connection portion 600.

In this state, the male connection portion 500 on the optical probe 300 side moves toward the female connection portion 600. As a result, in the lock mechanism 243, the proximal end of the longitudinal ridge 708 in the male lock portion 700 comes in contact with the distal surface of the annular protruding portion 824 of the female lock portion 800. In this state, after the male connection portion 500 is rotated to align the longitudinal ridges 708 respectively with the longitudinal grooves 826, the cylindrical outer circumferential surface 703 of the male lock portion 700 is pushed into the inner circumferential surface of the proximal cylindrical member 806 of the female lock portion 800. As a result, in the lock mechanism 243, the longitudinal ridges 708 respectively passes through the longitudinal grooves 826 and 846 to enter the recessed surfaces 844 at the proximal end.

In the process for pushing the male connection portion 500, the inclined end surface 536 of the male connection portion 500 comes in contact with the ridge 618 of the female connection portion 600. When the male connection portion 500 is moved toward the female connection portion 600 in this state, the ridge 618 relatively moves along the inclined end surface 536, and thus the distal rotation cylindrical portion 523 receives a circumferential rotation force. The rotation of the distal rotation cylindrical portion 523 is terminated when the ridge 618 reaches the deepest portion of the inclined end surface 536, and when, in this state, the male connection portion 500 is further moved toward the proximal end, the ridge 618 enters the guide portion 534. In this state, the connector 532 faces the adapter 610 in a connectable state. In the lock mechanism 243, the distal portions of the ridges 708 are respectively accommodated in the longitudinal grooves 846.

When the male connection portion 500 is further pushed toward the proximal end, the protrusions 704 of the lock mechanism 243 respectively enter the axially deepest portions of the circumferential recesses 842 and come in contact with radial walls (steps) 858 (see FIG. 7) between the radial bottom portions of the longitudinal grooves 846 at the proximal ends and the radial bottom portions of the circumferential recesses 842.

Next, when the male connection portion 500 is further pushed toward the proximal end against the urging forces of the springs 854, the protrusions 704 push the walls 858 to retract the proximal cylindrical member 806 toward the proximal end until the proximal end of the rib 857 of the proximal cylindrical member 806 comes in contact with the proximal end of the inner cylindrical portion 518. At this time, the connector 532 of the male connection portion 500 comes in contact with the adapter 610 and is urged toward the distal end. When the connector 532 is urged toward the distal end, the distal rotation cylindrical portion 523 moves to a retracted position where the distal end surface of the annular inner wall 524 comes in contact with the proximal end of the inner cylindrical portion 518.

At this time, the elastic member 530 contracts in accordance with the urging forces received by the connector 532 from the adapter 610. This prevents the connector 532 from excessively moving toward the distal end.

As described above, the connector 532 is urged toward the distal end with the connector being in contact with the adapter 610, and is connected to the adapter 610.

Next, when the male connection portion 500 is rotated (rotated in the clockwise direction when viewed from the distal end toward the proximal end in FIG. 7), the protrusions 704 respectively pass between the trapezoidal protrusions 828 and the circumferential walls 848 facing the trapezoidal protrusions to reach the circumferential end portions of the circumferential recesses 842. Next, when, this state, the force applied to the male connection portion 500 is released, the proximal cylindrical member 806 and the protrusions 704 are pushed back to the distal end by the forces of the springs 854. As a result, the protrusions 704 are respectively accommodated in the protrusion accommodating chambers 856, and the optical probe 300 is locked with the optical probe being connected to the rotation portion 238.

In a case where the optical probe 300 is separated from the rotation portion 238, the male connection portion 500 is pushed toward the proximal end against the urging forces of the springs 854, and thus the protrusions 704 retract the proximal cylindrical member 806 toward the proximal end. While this state is being maintained, the male connection portion 500 is rotated (rotated in the counterclockwise direction when viewed from the distal end toward the proximal end in FIG. 7). As a result, the protrusions 704 respectively pass between the trapezoidal protrusions 828 and the circumferential walls 848 facing the trapezoidal protrusions to reach between the longitudinal grooves 826 and 846. Next, when, in this state, the force applied to the male connection portion 500 is released, the protrusions 704 and the male connection portion 500 are pushed back to the distal end by the forces of the springs 854.

When the male connection portion 500 is moved to the distal end from the state where the protrusions 704 are respectively between the longitudinal grooves 826 and 846, the guide portion 534 is moved to the distal end, and the distal rotation cylindrical portion 523 is moved to the advanced position where the proximal end surface of the annular outer wall 528 comes in contact with the small-diameter cylindrical portion 522. As a result, the connector 532 is separated from the adapter 610. Further, in the lock mechanism 243, the longitudinal ridges 708 respectively move from the recessed surfaces 844 at the proximal end to the distal end through the longitudinal grooves 826 and 846. Finally, the male lock portion 700 is separated from the female lock portion 800 and the male connection portion 500 is separated from the female connection portion 600.

### Reference Signs List

- 238: drive portion (rotation portion)
- 242: connection mechanism
- 243: lock mechanism
- 300: optical probe
- 500: male connection portion
- 512: central axis
- 523: distal rotation cylindrical portion
- 532: connector
- 534: guide portion
- 536: inclined end surface
- 600: female connection portion
- 610: adapter
- 612: proximal rotation cylindrical portion
- 616: central axis
- 618: protruding portion
- 700: male lock portion
- 704: protrusion
- 800: female lock portion
- 802: distal cylindrical member
- 804: proximal fixing member
- 806: proximal cylindrical member
- 818: central axis
- 826: longitudinal groove
- 828: protrusion
- 842: circumferential recess
- 854: spring
- 856: space
- 858: wall

## Claims

1. A connection mechanism (242) that connects an optical probe (300) used in an optical tomographic imaging apparatus and a drive portion (238) that rotates the optical probe (300),
(a) the connection mechanism (242) comprising:
a female connection portion (600) having an approximately cylindrical shape disposed on a distal end side of the drive portion (238); and
a male connection portion (500) having an approximately cylindrical shape disposed on a proximal end side of the optical probe (300),
(b) wherein the female connection portion (600) includes
a proximal rotation cylindrical portion (612) having an approximately cylindrical shape disposed at a distal end of the female connection portion (600) coaxially with a central axis (616) of the female connection portion (600),
an adapter (610) fixed inside the female connection portion (600), and
a protruding portion (618) disposed on an inner circumferential surface of the female connection portion (600),
(c) wherein the male connection portion (500) includes
a distal rotation cylindrical portion (523) having an approximately cylindrical shape disposed at a proximal end of the male connection portion (500) coaxially with a central axis (512) of the male connection portion (500),
a connector (532) fixed inside the distal rotation cylindrical portion (523) correspondingly to the adapter (610),
a guide portion (534) disposed on an outer circumferential surface of the distal rotation cylindrical portion (523) and extending from an end portion of a proximal end of the distal rotation cylindrical portion (523) toward a distal end along the central axis (512) of the male connection portion (500), and
an inclined end surface (536) that is symmetrical with respect to a plane including the guide portion (534) and the central axis (512) and extends toward a proximal end along the central axis (512) of the male connection portion (500) at a proximal end of the distal rotation cylindrical portion (523),
the distal rotation cylindrical portion (523) being configured to rotate about the central axis (512) of the male connection portion (500), and
(d) wherein the male connection portion (500) is rotated by moving the male connection portion (500) toward the female connection portion (600) in a state where the distal rotation cylindrical portion (523) of the male connection portion (500) is inserted into the proximal rotation cylindrical portion (612) of the female connection portion (600) and the inclined end surface (536) of the male connection portion (500) is brought into contact with the protruding portion (618) of the female connection portion (600), the protruding portion (618) is inserted into the guide portion (534), and the connector (532) is connected to the adapter (610).

2. The connection mechanism (242) according to claim 1, wherein the distal rotation cylindrical portion (523) is configured to be movable along the central axis (512) of the male connection portion (500).

3. The connection mechanism (242) according to claim 1 or 2, wherein the male connection portion (500) includes an elastic member (530) that has an approximately cylindrical shape extending along the central axis (512) of the male connection portion (500) and is disposed between the male connection portion (500) and the connector (532).

4. A lock mechanism (243) that holds a connection between an optical probe (300) used in an optical tomographic imaging apparatus and a drive portion (238) that rotates the optical probe (300),
(a) the lock mechanism (243) comprising:
a female lock portion (800) having an approximately cylindrical shape disposed on a distal end side of the drive portion (238); and
a male lock portion (700) having an approximately cylindrical shape disposed on a proximal end side of the optical probe (300), the male lock portion (700) being configured to be inserted into the female lock portion (800),
(b) wherein the male lock portion (700) includes a first protrusion (704) disposed on an outer circumferential surface of the male lock portion (700),
(c) wherein the female lock portion (800) includes
a distal cylindrical member (802) having a substantially cylindrical shape fixed to the drive portion (238) coaxially with a central axis (818) of the female lock portion (800) at a distal end of the female lock portion (800),
a proximal fixing member (804) having an approximately cylindrical shape fixed to the drive portion (238) to face the distal cylindrical member (802) at a proximal end of the female lock portion (800),
a proximal cylindrical member (806) having an approximately cylindrical shape disposed between the distal cylindrical member (802) and the proximal fixing member (804), the proximal cylindrical member being movable along the central axis (818) of the female lock portion (800), and
a spring (854) that urges the proximal cylindrical member (806) toward a distal end,
wherein the distal cylindrical member (802) includes
a protrusion (828) disposed on an inner circumferential surface of the distal cylindrical member (802) and extending toward a proximal end of the female lock portion (800) along the central axis (818) of the female lock portion (800), and
a first longitudinal groove (826) disposed on one end in a circumferential direction of the protrusion (828), the first protrusion (704) of the male lock portion (700) passing through the first longitudinal groove (826),
wherein the proximal cylindrical member (806) includes a circumferential recess (842) having a shape corresponding to the protrusion (828) on an inner circumferential surface of the proximal cylindrical member (806) and having a circumferential length longer than the protrusion (828),
the circumferential recess (842) being configured to accommodate the protrusion (828) in a state where the proximal cylindrical member (806) being urged by the spring (854) and form a space (856) on a side opposing the first longitudinal groove (826) across the protrusion (828), and (d) wherein the first protrusion (704) of the male lock portion (700) is configured to, when the male lock portion (700) is inserted into the distal cylindrical member (802) of the female lock portion (800), pass through the first longitudinal groove (826) of the distal cylindrical member (802), come in contact with a wall (858) of the circumferential recess (842) disposed in the proximal cylindrical member (806) of the female lock portion (800), further, in this state, move the proximal cylindrical member (806) toward the proximal fixing member (804) against an urging force of the spring (854), and then rotate the male lock portion to move to the space (856) of the circumferential recess (842).

5. The lock mechanism according to claim 4,
wherein the proximal cylindrical member (806) includes an inner circumferential surface (841) having a recessed surface (844) disposed at a proximal end of the circumferential recess (842) with spacing away from the circumferential recess (842), a second longitudinal groove (846) connecting the circumferential recess (842) and the recessed surface (844) at one end of the circumferential recess (842), and a circumferential wall (848) surrounded on three sides by the circumferential recess (842), the recessed surface (844), and the second longitudinal groove (846),
wherein the male lock portion (700) includes a second protrusion (708) disposed on an outer circumferential surface in a position away from the first protrusion (704) toward a proximal end, and
wherein the second protrusion (708) passes through the second longitudinal groove (846) of the proximal cylindrical member (806) to be positioned on the recessed surface (844) in a state where the first protrusion (704) of the male lock portion (700) comes in contact with the wall (858), and when the male lock portion (700) in this state is rotated, the circumferential wall (848) enters between the first protrusion (704) and the second protrusion (708).
